# EUROPÄISCHE PATENTSCHRIFT

(11) **EP 2 794 546 B1**
(45) Veröffentlichungstag und Bekanntmachung des Hinweises auf die Patenterteilung: **03.02.2016**
(21) Anmeldenummer: 12790535.4
(22) Anmeldetag: 20.11.2012
(51) Int. Cl.: C07C 67/04, C07C 69/54

(54) **VERFAHREN ZUR HERSTELLUNG BI- ODER TRIZYKLISCHER (METH)ACRYLATE**
METHOD FOR PRODUCING BICYCLIC OR TRICYCLIC (METH)ACRYLATES
PROCÉDÉ POUR PRODUIRE DES (MÉTH)ACRYLATES BI- OU TRICYCLIQUES

(30) Priorität: 22.12.2011 DE 102011089504
(43) Veröffentlichungstag der Anmeldung: 29.10.2014
(73) Patentinhaber: Evonik Röhm GmbH, 64293 Darmstadt (DE)
(72) Erfinder: KNEBEL, Joachim, 64665 Alsbach-Hähnlein (DE); SCHÜTZ, Thorben, 64665 Alsbach-Hähnlein (DE); GRÄFF, Günther, 64665 Alsbach-Hähnlein (DE); OHL, Thomas, "verstorben" (DE)
(86) Internationale Anmeldenummer: PCT/EP2012/073087
(87) Internationale Veröffentlichungsnummer: WO 2013/092072

(56) Entgegenhaltungen:
- WO-A1-2008/023823
- HEIDEKUM A ET AL: "Addition of Carboxylic Acids to Cyclic Olefins Catalyzed by Strong Acidic Ion-Exchange Resins", JOURNAL OF CATALYSIS, ACADEMIC PRESS, DULUTH, MN, US, Bd. 181, Nr. 2, 25. Januar 1999 (1999-01-25), Seiten 217-222, XP004443248, ISSN: 0021-9517, DOI: 10.1006/JCAT.1998.2300

## Beschreibung

Die vorliegende Erfindung betrifft ein Verfahren zur Herstellung bi- oder trizyklischer (Meth)acrylate durch Umsetzung von (Meth)acrylsäure mit bi- oder trizyklischen Kohlenwasserstoffen, die mindestens eine Doppelbindung tragen. Die Schreibweise (Meth)acrylate betrifft sowohl Derivate der Methacrylsäure als auch Verbindungen der Acrylsäure.

Verfahren zur Herstellung von bizyklischen (Meth)acrylaten sind aus dem Stand der Technik bekannt. So beschreiben z.B. EP 718271 und Journal of Catalysis 181, 217-222 (1999) die Herstellung von Isobornyl(meth)acrylat ausgehend von Camphen und (Meth)acrylsäure an sauren Ionenaustauschern. EP 759423 beansprucht ein ähnliches Herstellverfahren unter Verwendung von Zirkonsulfat als Katalysator. Zwei Verfahren unter homogener Katalyse werden in DE 19920796 in Gegenwart von Schwefelsäure und in DE 4316004 in Anwesenheit von Molybdatophosphorsäure beschrieben. Ferner offenbart DE 19930721 zur Herstellung von Isobornyl(meth)acrylat ein Umesterungsverfahren ausgehend von Isoborneol und MMA bzw. Ethylacrylat.

Die genannten Verfahren weisen jedoch Nachteile auf. Zum Einen sind Katalysator (Zirkonsulfat) oder Ausgangsverbindung (Isoborneol) kommerziell nicht verfügbar. Zum Anderen sind thermische Stabilität und Lebensdauer des eingesetzten Katalysators (Ionenaustauscher) begrenzt und führen zum Austrag niedermolekularer, farbgebender Zersetzungsprodukte. Während seiner Lebensdauer lassen Aktivität und Selektivität nach. Zur Abtrennung der dadurch bedingten Nebenprodukte (Camphenoligomere) sind aufwendige Reinigungsschritte notwendig.

Aufgabe der vorliegenden Erfindung ist es daher ein alternatives Verfahren zur Herstellung bi- oder trizyklischer (Meth)acrylate zur Verfügung zu stellen, das die genannten Nachteile vollständig oder zumindest teilweise beseitigt. Gelöst werden diese sowie weitere im einzelnen nicht näher ausgeführte, jedoch aus der einleitenden Erörterung des Standes der Technik ohne weiteres erschließbare oder ableitbare Aufgaben durch ein Verfahren mit den Merkmalen des Patentanspruchs 1. Vorteilhafte Abwandlungen des erfindungsgemäßen Verfahrens werden in den auf Anspruch 1 rückbezogenen Ansprüchen unter Schutz gestellt.

Die vorliegende Erfindung betrifft ein Verfahren zur Herstellung von bi- oder trizyklischen (Meth)acrylaten durch Umsetzung von (Meth)acrylsäure mit einem bi- oder trizyklischen Kohlenwasserstoff, der mindestens eine Doppelbindung enthält, dadurch gekennzeichnet, dass als Katalysator Montmorillonit eingesetzt wird. Als anorganisches Material besitzt Montmorillonit gegenüber den im Stand der Technik eingesetzten Katalysatoren den Vorteil der höheren thermischen Stabilität im Vergleich zu den polystyrolbasierten Kationenaustauschern, der Abwesenheit farbgebender Zersetzungsprodukte und der kommerziellen Verfügbarkeit. Ferner läßt er sich durch einen relativ einfachen Filtrationsschritt aus der Reaktion entfernen und ist überraschenderweise ohne Nachteile mehrfach für die gleiche Umsetzung verwendbar.

Im erfindungsgemäßen Verfahren lassen sich im Prinzip alle bi- oder trizyklischen Kohlenwasserstoffe, die mindestens eine Doppelbindung besitzen, mit (Meth)acrlysäure umsetzten. Beispielhaft seien genannt als bizyklische Kohlenwasserstoffe Pinen, Camphen, Fenchen, Isobornylen, Norbornen, Isocamphen,Thujen, Sabinen, Caren und Santalen, als trizyklische Kohlenwasserstoffe Dicyclopentadien, Gurjunen, Aristolon, Cedren und Bourbonen oder solche Tricycloalkene, wie sie sich z.B. nach US 3,725,366 als dortige Ausgangsverbindungen herstellen lassen. Bevorzugt eingesetzt werden Camphen, Norbornen oder Dicyclopentadien.

Als Katalysator wird Montmorillonit eingesetzt. Montmorillonit, so benannt nach seiner Lagerstätte bei Montmorillon in Südfrankreich, wo das Mineral in Europa erstmals entdeckt wurde, ist ein häufig vorkommendes Mineral aus der Gruppe der Tonminerale innerhalb der Mineralklasse der Silikate und Germanate. Es kristallisiert im monoklinen Kristallsystem mit der chemischen Zusammensetzung ~(Al_{1,67}Mg_{0,33})[(OH)₂|Si₄O₁₀] · Na_{0,33}(H₂O)₄ und entwickelt mikroskopisch kleine, nadelige Kristalle, die gewöhnlich kompakte, massige Aggregate bilden. Montmorillonit besitzt ein hohes Ionenaustauschvermögen, da er die Kationen zwischen den Schichten gegen die in Lösung befindlichen austauschen kann. Bei Zugabe von Wasser dehnt sich das Mineral um ein vielfaches der Ursprungsgröße aus. Die seit 2001 gültige und von der International Mineralogical Association (IMA) verwendete 9. Auflage der Strunz'schen Mineralsystematik ordnet den Montmorillonit in die Abteilung der Schichtsilikate (Phyllosilikate) ein. Diese Abteilung ist weiter unterteilt nach der Kristallstruktur, so dass das Mineral entsprechend seines Aufbaus in der Unterabteilung der Schichtsilikate (Phyllosilikate) mit Glimmertafeln, zusammengesetzt aus tetraedrischen oder oktaedrischen Netzen, zu finden ist, wo es als Namensgeber die Montmorillonitgruppe mit der System-Nr. 9.EC.40 bildet. Weitere Einzelheiten zu Montmorillonit sind bei Hugo Strunz, Ernest H. Nickel: Strunz Mineralogical Tables. 9. Auflage. E. Schweizerbart'sche Verlagsbuchhandlung (Nägele u. Obermiller), Stuttgart 2001, ISBN 3-510-65188-X nach zu lesen. Im erfindungsgemäßen Verfahren wird bevorzugt eine natürlich vorkommende Variante des Montmorillonit, der nach seinem Fundort in Benton, Wyoming, USA benannte Bentonit eingesetzt. Bentonit ist die Bezeichnung für ein Industriemineral, welches neben dem Hauptmineral Montmorillonit noch Begleitmineralien wie Quarz, Glimmer, Feldspat, Pyrit oder auch Kalk enthält. Dieses Produkt ist unter der Bezeichnung Montmorillonit K10 bei Südchemie kommerziell erhältlich. Die bei der Umsetzung eingesetzten Mengen an Montmorillonit bewegen sich zwischen 5-50 Gew%, bevorzugt zwischen 10-40 Gew% und besonders bevorzugt zwischen 15-30 Gew% bezogen auf die eingesetzte Menge an (Meth)acrylsäure.

Im erfindungsgemäßen Verfahren wird eine polymerisierbare Verbindung umgesetzt. Daher muss die Reaktion in Anwesenheit von Polymerisationsinhibitoren erfolgen. Zu den bevorzugt einsetzbaren Polymerisationsinhibitoren gehören unter anderem Phenothiazin, Tertiärbutylcatechol, Hydrochinonmonomethylether, Hydrochinon, 4-Hydroxy-2,2,6,6- tetramethylpiperidinooxyl (TEMPOL) oder deren Gemische, wobei die Wirksamkeit dieser Inhibitoren durch Einsatz von Sauerstoff teilweise verbessert werden kann. Die Polymerisationsinhibitoren können in einer Konzentration im Bereich von 0,001 bis 2,0 Gew%, besonders bevorzugt im Bereich von 0,01 bis 0,2 Gew%, bezogen auf (Meth)acrylsäure, eingesetzt werden. Die Reaktion kann kontinuierlich oder batchweise in dem Fachmann geläufigen Anlagen erfolgen. Es bieten sich CSTR oder Rohrreaktoren an, bevorzugt findet die Reaktion in einem CSTR statt. Der Katalysator wird über übliche Filtrationsschritte entfernt und kann optional für die Reaktion wiederverwendet werden. In der Regel ist ein destillativer Aufreinigungsschritt anzuschließen.

Die erfindungsgemäße Umsetzung findet unter Normaldruck oder leicht erhöhtem Druck, bevorzugt zwischen 1-10 bar, besonders bevorzugt zwischen 1-5 bar statt. Die Reaktionstemperaturen liegen hierbei zwischen 50-250 °C, bevorzugt zwischen 70-200 °C und besonders bevorzugt zwischen 90-160 °C. Die Verweilzeiten in Batchfahrweise betragen in der Regel 5-30 h, bevorzugt 5-20 h, besonders bevorzugt 5-10 h. Der anschließende optional destillative Aufreinigungsschritt orientiert sich an der Zusammensetzung des erhaltenen Reaktionsgemisches an Produkten und Edukten und deren Siedepunkte, und wird in einer dem Fachmann geläufigen Apparatur, in der Regel mindestens einer Trennkolonne, und Verfahrensweise durchgeführt.

Die erfindungsgemäß erhaltenen (Meth)acrylate können als Monomere in an sich bekannter Weise in Lackharzen oder strahlungshärtbaren Beschichtungen eingesetzt werden.
Die folgenden Beispiele sollen die Erfindung erläutern, aber in keiner Weise einschränken.

### Beispiel 1

In einen 500 ml Vierhalsrundkolben, enthaltend Säbelrührer mit Rührhülse, Thermometer, Rückflußkühler, Lufteinleitrohr sowie temperaturgeregeltes Ölbad werden 66,3 g (0,77 mol) Methacrylsäure, 95,4 g (0,70 mol) Camphen, 0,078 g Hydrochinonmonomethylether als Polymerisationsinhibitor sowie 16,9 g Montmorillonit K10 (Südchemie) eingewogen. Unter Rühren und Lufteinleiten erhitzt man 6 h auf 90 °C. Man lässt abkühlen, filtriert vom Montmorillonit ab und analysiert das Filtrat gaschromatographisch. Der Katalysator wird in den Folgebeispielen wieder eingesetzt. Die GC-Analytik ergibt 10,9 % unumgesetzte Methacrylsäure, 20,5 % unumgesetztes Camphen, 60,3 % Isobornylmethacrylat sowie 6,3 % Camphendimer und 0,6% Isoborneol.

### Beispiele 2-7

In den Beispielen 2-7 wird jeweils Beispiel 1 wiederholt mit Einsatz des Katalysators aus dem jeweils vorhergehenden Ansatz. Die Zusammensetzung der erhaltenen Reaktionsgemische ist in Tab. 1 dargestellt.

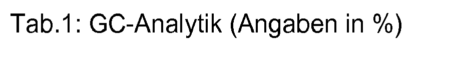

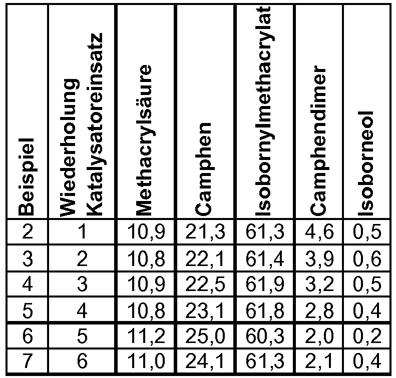

Wie aus Tab. 1 zu ersehen, birgt die Wiederverwendung des Katalysators keinerlei Nachteile, überraschenderweise verbessert sich die Zusammensetzung bezüglich der nicht rezyklierbaren Nebenprodukte.

### Beispiel 8

In einen 500 ml Vierhalsrundkolben, enthaltend Säbelrührer mit Rührhülse, Thermometer, Rückflußkühler, Lufteinleitrohr sowie temperaturgeregeltes Ölbad werden 66,3 g (0,77 mol) Methacrylsäure, 92,4 g (0,70 mol) Dicyclopentadien, 0,078 g Hydrochinonmonomethylether als Polymerisationsinhibitor sowie 17 g Montmorillonit K10 (Südchemie) eingewogen. Unter Rühren und Lufteinleiten erhitzt man 6 h auf 90 °C. Man lässt abkühlen, filtriert vom Montmorillonit ab und analysiert das Filtrat gaschromatographisch. Der Katalysator kann optional wieder verwendet werden. Die GC-Analytik ergibt 11 % unumgesetzte Methacrylsäure, 22 % unumgesetztes Dicyclopentadien, 60 % Dicyclopentenylmethacrylat-Isomerengemisch sowie insgesamt 6 % Dicyclopentadienoligomere und 0,6% Bis(dicyclopentenyl)ether.

## Patentansprüche

1. Verfahren zur Herstellung von bi- oder trizyklischen (Meth)acrylaten durch Umsetzung von (Meth)acrylsäure mit einem bi- oder trizyklischen Kohlenwasserstoff, der mindestens eine Doppelbindung enthält, **dadurch gekennzeichnet, dass** als Katalysator Montmorillonit eingesetzt wird.

2. Verfahren nach Anspruch 1 **dadurch gekennzeichnet, dass** der Katalysator in Mengen zwischen 5-50 Gew%, bezogen auf (Meth)acrylsäure, eingesetzt wird.

3. Verfahren nach Anspruch 1 **dadurch gekennzeichnet, dass** als bizyklischer Kohlenwasserstoff Camphen, Norbornen oder Dicyclopentadien eingesetzt werden.

4. Verfahren nach Anspruch 1 **dadurch gekennzeichnet, dass** Bentonit als Katalysator eingesetzt wird.

5. Verfahren nach Anspruch 1 **dadurch gekennzeichnet, dass** die Reaktion bei 50-250 °C durchgeführt wird.

## Claims

1. Process for preparing bicyclic or tricyclic (meth)acrylates by reacting (meth)acrylic acid with a bicyclic or tricyclic hydrocarbon which contains at least one double bond, **characterized in that** montmorillonite is used as catalyst.

2. Process according to Claim 1, **characterized in that** the catalyst is used in amounts in the range 5-50% by weight, based on (meth)acrylic acid.

3. Process according to Claim 1, **characterized in that** camphene, norbornene or dicyclopentadiene is used as bicyclic hydrocarbon.

4. Process according to Claim 1, **characterized in that** bentonite is used as catalyst.

5. Process according to Claim 1, **characterized in that** the reaction is carried out at 50-250°C.

## Revendications

1. Procédé de fabrication de (méth)acrylates bi- ou tricycliques par mise en réaction d'acide (méth)acrylique avec un hydrocarbure bi- ou tricyclique qui contient au moins une double liaison, **caractérisé en ce qu'**une montmorillonite est utilisée en tant que catalyseur.

2. Procédé selon la revendication 1, **caractérisé en ce que** le catalyseur est utilisé en quantités comprises entre 5 et 50 % en poids, par rapport à l'acide (méth)acrylique.

3. Procédé selon la revendication 1, **caractérisé en ce que** du camphène, du norbornène ou du dicyclopentadiène est utilisé en tant qu'hydrocarbure bicyclique.

4. Procédé selon la revendication 1, **caractérisé en ce qu'**une bentonite est utilisée en tant que catalyseur.

5. Procédé selon la revendication 1, **caractérisé en ce que** la réaction est réalisée à 50 à 250 °C.
